# EUROPEAN PATENT SPECIFICATION

(11) **EP 1 171 144 B1**
(45) Date of publication and mention of the grant of the patent: **14.02.2007**
(21) Application number: 00921582.3
(22) Date of filing: 07.04.2000
(51) Int. Cl.: A61K 36/9066, A61K 31/12, A61P 17/00, A61P 35/00

(54) **USE OF TETRAHYDROCURCUMINOIDS TO REGULATE PHYSIOLOGICAL AND PATHOLOGICAL EVENTS IN THE SKIN AND MUCOSA**
VERWENDUNG VON TETRAHYDROCURCUMINOIDEN ZUR REGULATION VON PHYSIOLOGISCHEN UND PATHOLOGISCHEN FÄLLEN IN DER HAUT UND MUCOSA
UTILISATION DE TETRAHYDROCURCUMINOIDES POUR LA REGULATION D'EVENEMENTS PHYSIOLOGIQUES ET PATHOLOGIQUES AU NIVEAU DE LA PEAU ET DES MUQUEUSES

(30) Priority: 09.04.1999 US 128540 P
(43) Date of publication of application: 16.01.2002
(73) Proprietor: Sabinsa Corporation, Piscataway, NJ 08854 (US); Sami Chemicals and Extracts (P) Ltd., Peennya, Bangalore 560 058 (IN)
(72) Inventor: MAJEED, Muhammed, Piscataway, NJ 08854 (US); BADMAEV, Vladimir, Piscataway, NJ 08854 (US)
(74) Representative: Weiss, Wolfgang
(86) International application number: PCT/US2000/008711
(87) International publication number: WO 2000/061162

(56) References cited:
- WO-A-95/18606
- WO-A-97/03674
- JP-A- 6 128 133
- US-A- 5 266 344
- OSAWA, TOSHIHIKO ET AL: "Antioxidative activity of tetrahydrocurcumin" INT. CONGR. SER. - EXCERPTA MED. (1992), 998(OXYGEN RADICALS), 801-4 , XP000933940
- WALKER, M. J. ET AL: "Curcumin in a chemoprevention model of melanoma." PROCEEDINGS OF THE AMERICAN ASSOCIATION FOR CANCER RESEARCH ANNUAL MEETING, (MARCH, 1998) VOL. 39, PP. 19. MEETING INFO.: 89TH ANNUAL MEETING OF THE AMERICAN ASSOCIATION FOR CANCER RESEARCH NEW ORLEANS, LOUISIANA, USA MARCH 28-APRIL 1, 1998 AMERICAN , XP000929727
- RUBY, A. J. ET AL: "Antitumor and antioxidant activity of natural curcuminoids" CANCER LETT. (SHANNON, IREL.) (1995), 94(1), 79-83 , XP000933937
- MENON L G ET AL: "Anti-metastatic activity of curcumin and catechin." CANCER LETTERS, (1999 JUL 1) 141 (1-2) 159-65. , XP000933941
- MENON L G ET AL: "Inhibition of lung metastasis in mice induced by B16F10 melanoma cells by polyphenolic compounds." CANCER LETTERS, (1995 AUG 16) 95 (1-2) 221-5. , XP000934170
- MULKY, N. ET AL: "Antimutagenicity of curcumins and related compounds: the structural requirement for the antimutagenicity of curcumins" INDIAN DRUGS (1987), 25(3), 91-5 , XP000934197
- NAGABHUSHAN, M. ET AL: "Curcumins as inhibitors of nitrosation in vitro" MUTAT. RES. (1988), 202(1), 163-9 , XP000933930
- SHARMA, O. P.: "Antioxidant activity of curcumin and related compounds" BIOCHEM. PHARMACOL. (1976), 25(15), 1811-12 , XP000933938

## Description

### Background of the invention

A body cell owes its functioning in large part to its cytoskeleton, the structural framework of the cell. The cytoskeleton is formed by components in the cytoplasm that maintain cell shape, stabilize cell attachments to neighboring cells, and facilitate specialized cell functions like endocytosis, exocytosis and cell motility. The cytoskeleton includes several filamentous structures like microtubules, microfilaments, intermediate filaments, and microtrabecular lattice. Proteins participating in the formation of a cytoskeleton, especially microtubular-associated proteins (MAPS) after being manufactured by the cell, are further adapted to the cytoskeleton function in a process called post-translational modification, or cross-linking. This process of proteins cross-linking is essential to stabilizing the cytoskeleton, the event necessary to maintain cell homeostasis, physiological processes, and cell development and maturation.

The post-translational modification of proteins involves several chemical reactions. Protein phosphorylation exemplifies one of the most common mechanisms of post translation modification of cellular proteins. Other examples include methylestrification of proteins, ADP-ribosylation, protein glycosylation, histone acetylation, hypusine formation and transglutaminase-catalyzed reactions.

The electric potential of a cell is an aspect of cellular physiology important to the understanding of the mechanism of the invention. The electric potential of a cell is maintained by a complex balance between negatively and positively charged molecules which results in a physiologic resting potential inside the cell of approximately - 70 mV. This value is indicative of the cell's well-being. On the other hand, the inability of a cell to maintain an optimal resting potential occurs in pathological conditions, e.g. physical or chemical assaults, a disease process, and the wear and tear due to aging. Optimal resting potential is conductive to the physiological processes including post-translational protein modification and cross-linking. It also holds true that the intact physiological cross-linking of proteins is indicative of a cell's ability to maintain optimal resting electric potential,

### Positive effects of cross-linking

Cross-linking of proteins, as it has been previously mentioned, is an outcome of the post translational modification of proteins. Some of the most common cross-links identified in proteins forming cytoskeleton are Neee-(ggg-glutamyl)lysine and N,N-bis(gggglutamyl)polyamine. The formation of these two types of cross-links is facilitated by the catalytic activity of a group of enzymes known as transglutaminases. One of the well known transglutaminases in the body is coagulation protein Factor XIII. This Factor causes cross-linking of the fibrin, which acts as a stabilizing agent on blood clot formation. This type of modification is an example of a life saving mechanism-based on protein cross-linking, that prevent exsanguination in a cut or a wound.

Transglutaminases are responsible for facilitating a physiological process of protein cross-linking, which under normal conditions leads to cell differentiation, thus the cell is able to fulfill its purpose, e.g. mucus secreting cell, phagocytic cell, epithelial and tegumen cell protecting the tissue structure. Transglutaminase activity, or its absence, plays a critical role in the differentiation of cancer cells, especially skin cancer melanoma. Melanoma is a rapidly spreading cancer consisting of undifferentiated skin cells, and the degree of poor cell differentiation correlates well with the suppressed activity of transglutaminase. On the other hand, experimental induction of transglutaminase activity led to promising results in slowing-down or reversing melanoma progression. Examples of transglutaminase inducers are retinoic acid and methylxantines. It appears that the therapeutic potential of retinoids in melanoma is related to their ability to activate intracellular transglutaminases.

### Detrimental cross-linking

Cross-linking of intracellular proteins is, however, not always a positive event. When a random and massive cross-linking is precipitated by an abrupt, non-physiological stimulus, an accelerated aging of the cell occurs. Due to the nature of random crosslinking, which defies the purpose of cell organization and differentiation, this may lead not only to premature cell aging and death, but also can lead to a pathology including malignancy. For example, the UVA-generated free radicals in skin cells can cause premature cross-linking of proteins and liberation of proteolytic enzymes. This latter event leads to the destruction of the cytoskeleton, and the rapid deterioration of cell organization which compromises cell functioning.

It is of special interest to mention here the term lipofuscin, nicknamed "the aging pigment" or "liver spots", which occurs in the epidermis and mucous membranes probably as a result of disrupted cellular and systemic metabolism. Lipofuscin has been shown to result from the oxidative degeneration of mitochondria and/or lysosomes in the cells. Lipofuscin is postulated to be a result of a metabolic error which results in hypoanabolism combined with hyperkatabolism. It is postulated here that lipofuscin formation is in part a manifestation of the disrupted process of post-translational protein modification, and that this process intensifies with any systemic disease process, including the aging process.

### Related art

As previously mentioned, transglutaminases are responsible for facilitating the physiological process of protein cross-linking, which under normal conditions leads to cell differentiation, maturation and timely aging. There is considerable evidence that intracellular transglutaminase activity may be inhibited in certain pathology, for example malignancy, e.g. melanoma. It appears that therapeutic potential of transglutaminase inducers like retinoids and methylxantines in melanoma is related to their ability to activate intracellular transglutaminases. This in turn would lead to differentiation of cells, which in clinical practice would lead to a slow down of the disease process due to a decrease in the number of undifferentiated malignant cells.

Curcumin derivatives including tetrahydrocurcumin and curcuminoids are well recognized in literature as a group of phenolic antioxidants (Majeed et al. Curcuminoid book). Majeed et al., U.S. Patent No. 5,861,415 established that the combination of naturally occurring curcuminoids isolated from turmeric root, i.e. curcumin, demethoxy curcumin and bisdemethoxy curcumin produce a broad range of antioxidant protection described as bioprotectant action. Bioprotectant action, characterized by the prevention of free radical formation and intervention in scavenging free radicals, is exerted by tetrahydrocurcumin(oids) (THC) as well as curcumin(oids) (Majeed et al., *Tumeric and the Healing Curcuminoids,* Keats Publications 1996 and Majeed et al., *Curcuminoids Antioxidant Phytonutrients,* NutriScience Publishers 1995). However, tetrahydrocurcumin(oids) show superior free radical scavenging properties, as compared to curcumin or curcuminoids (Majeed et al. Curcuminoid book). Another advantage of tetrahydrocurcuminoids is that they are not as highly colored curcuminoids and thus can be applied topically without significant staining.

Recently curcumin but not tetrahydrocurcumin was found to have a preventive effect on cross-linking of collagen in diabetic rats. (Sajithlal GB, Chithra P, Chandrakasan G., Biochem Pharmacol 1998 Dec 15;56(12):1607-14). However, the anti cross-linking effect of curcumin was attained with a very high oral dose of the compound (200 mg/kg body wt.). It was also noted that the preventive effect of curcumin on the advanced cross-linking of collagen was more pronounced than its therapeutic effect. The authors of this study explained that the anti cross-linking mechanism of curcumin is due to quenching the free radicals, which in turn would prevent the accelerated cross-linking of collagen in diabetes. This study supports the use of curcumin to prevent and to some degree treat excessive cross-linking of proteins in conditions characterized by accelerated cross-linking such as diabetes.

### Description of the invention

The invention is suitable for a method for treating a premalignant state or a malignancy in a patient comprising administering an effective amount of a mixture of tetrahydrocurcuminoids to a patient suffering from a premalignant state or a malignancy, in particular for a method ; wherein said malignancy is melanoma.

Therefore one aspect of the invention is the use of an effective amount of a mixture of tetrahydrocurcuminoids for the manufacture of a medicament for treating melanoma.

The invention is also suitable for a method for reducing "age spots" due to lipofuscin formation in a patient in need of such treatment, comprising administering an effective amount of a mixture comprising 75-85% tetrahydrocurcumin, 10-20% tetrahydrodemethoxy curcumin, and 2.0-4.5% tetrahydrobisdemethoxy curcumin to said patient, in particular for a method wherein, said composition is administered topically.

Therefore, a further aspect of the invention is the use of an effective amount of a mixture of tetrahydrocurcuminoids for the manufacture of a medicament for the treatment of age spots due to lipofuscin formation.

The mechanism of the invention is in preserving homeostasis and functioning of cells based on its regulatory, both inhibitory and stimulating, effect on the cross-linking of intracellular proteins. This regulatory mechanism, previously unknown for curcumin and its derivatives, is also unrelated to the well researched and described antioxidant properties of curcumin(oids) and tetrahydrocurcumin(oids). It is a novel way of accomplishing a broad regulatory effect on biological processes. A comparable regulatory effect on cross-linking of proteins in the organism was not described previously with any single compound or with compounded products. In essence the invention differs from other compounds affecting the cross-linking process in that the previously described compounds would stimulate or inhibit intracellular cross-linking, while the invention would act as a cross-linking modifier (CLM) or "crossregulin". These terms are used here for the first time to describe the mechanism of the invention. CLM or crossregulin indicates that a composition of the present invention exerts a positive influence on the post-translational protein modification and cross-linking process in both physiological and pathological conditions alike. The CLM effect of the invention is accomplished by using a specific composition of the product as described in the section "Process of Obtaining THC". The mechanism also occurs with modifications of the THC molecule, for example ether THC, trimethyl ether THC, dimethyl ether THC, benzyl ether THC and potassium, magnesium and copper salts of THC. The mechanism of the invention has been demonstrated in *in vivo* experimental conditions such as preventing UV sunburn and cross-linking (percent of thymine dimer cells) in the skin cells exposed to UV radiation. The biological properties of the invention were compared with those of curcuminoids, and despite the fact that the present composition is devoid of yellow color it still produces comparable protective effects against UV radiation as that of yellow curcuminoids. This activity appears to be different from the antioxidant effects characteristic of THC and curcuminoids.

**The following describes the mechanism of the invention in detail:**
1. It is proposed that in physiologic states characterized by optimal post-translational protein modification, protein cross-linking, as well as the optimal cell electric potential, THC would not interfere with the above exemplified states of cellular well-being.
2. However, in the states, characterized by poor morphological and functional differentiation of cells, e.g. pre-malignant states or malignancy, THC would contribute to protein post-translational modification and cross-linking and optimization of cell electric potential. This would be accomplished by interaction between phenolic rings and olefinic bonds of THC and NH2 as well as COO groups of the protein amino acids. The resulting bonds would act as an "anchor" that would initiate cross-linking with other proteins.
3. In the states of cell aging and in the states where random cross-linking is precipitated by a thermal injury due to the action of UV rays, for example, the undesired process would be prevented by the invention. THC would prevent undesired cross-linking by attaching to amino and carboxy groups of the protein amino acids. This would result in interference with the random cross-linking, a mechanism comparable to a "buffering" action. This "buffering" action would effectively stop the chain reaction which otherwise would lead to deterioration of cell homeostasis, premature aging and premature cell death.
4. The invention also has a supportive mechanism in preventing random cross-linking of intracellular proteins which is based on the antioxidant properties of THC, specifically on the invention's ability to scavenge free-radicals. It is proposed that due to this antioxidant action it would facilitate disruption of the electric charges of "supercharged" protein molecules, which is one of the reasons why random cross-linking is accelerated. This combined mechanism of crossregulin and antioxidant action is particularly applicable in preventing "age spots" due to lipofuscin formation, and inflammatory conditions such as psoriasis.
5. The source of oxygen moieties in THC for binding to NH2 or COO groups would be from the para hydroxy group no longer in conjugation with the olefinic bond, and from the hydroxy groups of phenolic rings.
6. Unlike the curcumin(oid) compound(s), THC molecule(s) can rotate more freely, thus providing better access to the reactive groups for "anchor" and/or "buffering" reactions.
7. Whereas, as previously mentioned, the invention is relatively inactive in states of cell physiology, its crossregulin mechanism would be gradually expressed proportionate to the degree of stress acting upon the cell. Thus the crossregulin mechanism of THC in the biological system would depend on the kind of injurious action experienced by the biological system.

### Process of obtaining THC:

Tetrahydro curcuminoids of the invention are products of the chemical reduction of curcuminoids, obtained from the rhizomes of *Curcuma longa,* commonly known as turmeric, or any other suitable plant from the botanical family Zingiberaceae.

The prior art teaches that when curcuminoids obtained from *Curcuma longa* are catalytically reduced and then isolated in the process of crystallization, tetrahydrocurcumin (THC) is recovered without a loss, but with substantial losses of tetrahydrobisdemethoxy curcumin (THBDC) and tetrahydrodemethoxy curcumin (THDC). As a result, the ratio among tetrahydrocurcuminoids is significantly changed as compared to tile original ratio of parent compounds, curcuminoids. The ratio of curcuminoids is preferred, because it correlates with the optimal biological activity of curcuminoids. U.S. Patent No. 5,861,415 describes curcuminoids in a specific and optimal ratio as Bioprotectants, providing maximal protective effect of the living cell.

The present invention provides a new, more efficient method of recovering THCs. This process prevents the loss of TBDMC and TDMC, and provides a Bioprotectant composition of THCs.

| THCurcuminoids | % content prior art | % content invention |
|---|---|---|
| Tetrahydro curcuminoids | 90 | 75-85 |
| Tetrahydro demethoxy curcumin | 9 | 10-20 |
| Tetrahydro bisdemethoxy curcumin | 1 | 2 - 4.5 |

### Brief outline of the prior art process for producing THCs

| | |
|---|---|
| Natural curcuminoids (95 %) | - 100 gm |
| Acetone | - 1 liter |
| Pd-C (catalyst) 5% | - 2.5 gm |

The solution of natural curcuminoids was charged into a hydrogenator. The catalyst was added carefully and the pressure of hydrogen was maintained at about 2 Kg/cm2. This reaction is completed in 3 to 4 hrs. The catalyst is then filtered out and removed. The acetone solution is concentrated under vacuum. The residue is crystallized from toluenes. The tetrahydro compounds are crystallized out as a creamy white powder having a melting point of 92-94°C.

### Process of the Present invention

This process utilizes a novel method of saturation of two olefinic bonds in curcuminoids in the catalytic hydrogen transfer reaction known as a hydride transfer reaction. The following is an example of a hydride transfer method.
1. A 3 liter round bottom flask with a stirrer and a reflux condenser is charged with 1 liter ethyl acetate;
2. The solution is charged with 250 gm of natural curcuminoids in a previously described Bioprotectant composition, i.e. curcumin (C) 70-80%, Demethoxy curcumin (DMC) 15-20% and Bisdemethoxy curcumin (BDMC 2.5-6.5% and stirred to uniformity at room temperature;
3. This mixture is charged with 1 liter of triethylamine followed by 12 gm of Palladium carbon and 80 ml of formic acid;
4. The mixture was stirred and subsequently refluxed at 80 deg. Celsius for 12 hours.
5. Periodically formic acid in 5 ml aliquots was charged at 2 hours intervals while a reflux was carried on;
6. TLC was checked to monitor the completion of the reaction. (system: Silica Precoated 0.25 mm thick plates/chloroform : methanol = 9:1);
7. After completion of the reaction, the reaction mixture was cooled to room temperature. It was filtered to remove palladium catalyst and washed with 50 ml of ethyl acetate;
8. Ethyl acetate and triethylamine were distilled off (2 liters of distillate was collected);
9. The crude mass was then extracted with toluene (3 x 500 ml);
10. The toluene extract was washed with 200 ml of 5% HCl and 3 x 500 ml of water and dried over sodium sulphate;
11. The solvent was removed under vacuum to get a pale yellow paste approximately 200 gm.
12. The paste was slurred with 200 ml of ether, filtered and dried under vacuum to obtain a pale yellow powder of tetrahydrocurcuminoids, Yield 175 gm (70%).

### Assay:

| | |
|---|---|
| THC | 75 to 85% |
| THDHC | 10 to 20% |
| THBDMC | 2 to 4.5% |

In another variation of hydride transfer reaction, curcuminoids were treated with ammonium formate or sodium dihydrogen phosphite (as a catalytic hydrogen transfer reagent) in the presence of Pd/C and acetic acid. The reduction is complete in 20 to 30 minutes at 100 to 110 Celsius degrees. This method yields 50% of tetrahydrocurcuminoids.

THC can be administered topically in a suitable vehicle or with an application device, e.g. a transdermal patch, in a concentration ranging from 0.025% to 5%; in the form of subcutaneous injections or a transdermal patch in a concentration ranging from 0.025% to 5%; or in orally administered dosage form in a concentration ranging from 5 mg to 500 mg per dose or 0.083 - 8.3 mg/kg of body weight. THC is suitable for human and animal use.

Other routes of administration include but are not limited to: aerosol or other device for delivery to the lungs, nasal spray, intravenous, intramuscular, intraperitoneal, intrathecal, vaginal, and rectal. Excipients which can be used as a vehicle for the delivery of the phage will be apparent to those skilled in the art.

Compositions of tetrahydrocurcuminoids for topical administration should be essentially colorless. The term "essentially colorless" is intended to mean that the composition contains no color or such a small amount of color that the composition does not stain the skin upon topical administration.

The term "color removed curcumin" refers to the tetrahydrocurcuminoid mixture according to the present invention.

THC molecule acting as an "anchor" for amino acids of proteins, to initiate protein cross-links. This mechanism would contribute to the physiological cross-links.

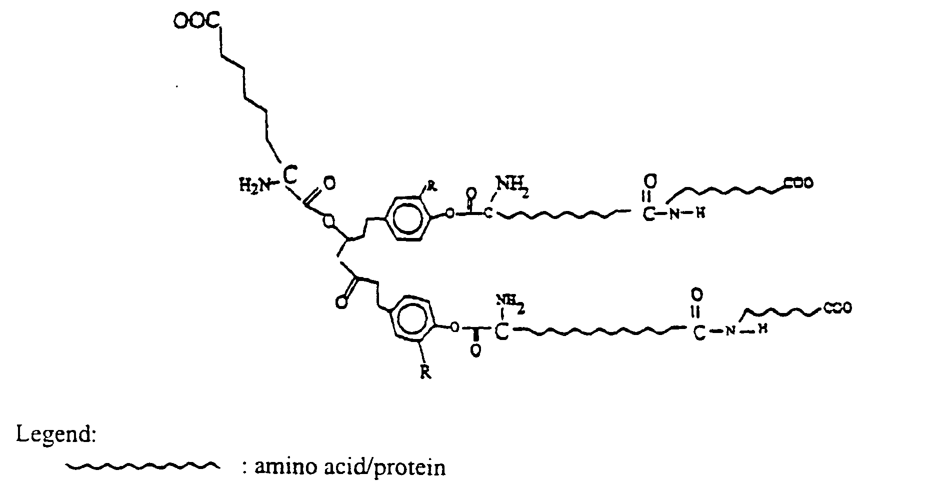

THC molecule acting as a buffer in preventing random cross-links in UV precipitated cross-links. This mechanism would contribute to the prevention of pathological cross-links.

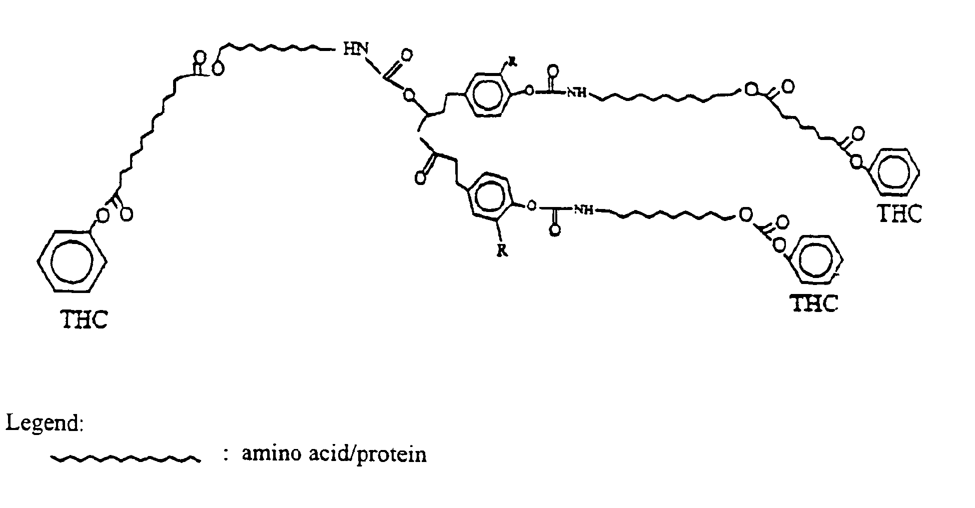

### INTRODUCTION

The study was designed to determine the acute oral toxicity of **Colour Removed Curcumin** to Sprague Dawley rats. The guidelines followed were as prescribed by the Gaitonde Committee Guidelines, CIB, New Delhi.

### PROJECT NO. 4952

### MATERIALS AND METHODS

### TEST SUBSTANCE:

| | |
|---|---|
| Sponsor : | **Sami Chemicals and Extracts Ltd., Bangalore.** |
| Label on Sample : | **Colour Removed Curcumin** Batch No.- CRC/65 A R No. - BL8C 0445 |
| Description : | Creamy white crystalline powder |
| Identification : | IR Spectrum: Complies |
| Solubility : | Soluble in methanol |
| Purity by HPLC : | 99.0% |

### SUMMARY

The study now reported was designed to determine the acute oral toxicity of Colour Removed **Curcumin** to Sprague Dawley rats. The test substance suspended in corn oil was administered by oral route to rats. The rats were observed for 14 days after treatment for the product related symptoms. The test substance caused salivation in all animals and polyurea in two animals with onset at 2 hours after the treatment. All animals survived through the study period of 14 days and were free of intoxicating signs 24 hours after the treatment.
The LD₅₀ value of **Colour Removed Curcumin** in rats by oral route was found to be greater than 5000 mg/kg.

### TEST SYSTEM

- Species: : Rat
- Strain: : Sprague Dawley
- Source: : I.I.T. Animal house
- Sex: : Male and Female
- Age: : 6 to 8 weeks
- Weight range: : 120.5 to 141.9 gms
- No. of animals per dose: : 5 per sex
- Randomization: : Randomly selected in groups of five of like sex at the time of initiating the study.
- Acclimation: : Not done as the animals were bred and reared in the same Institute.
- Identification of animals: : By cage number and individual marking on fur.
- Diet: : Pelleted feed supplied by Nav Maharashtra Chakan Oil Mills Ltd., Pune.
- Water: : Aquaguard pure water in glass bottles *ad libitum.*
- Management: : The rats were housed 5 each, of the same sex in polypropylene cages provided with bedding of husk. The temperature was maintained between 20 & 24 °C and relative humidity between 50 and 60%; 12 hours each of dark and light cycle was maintained.
- Route: : Oral gavage
- Vehicle used: : Corn oil
- Dose volume: : 10 ml/kg

### EXPERIMENTAL PROCEDURE

The test substance, suspended in corn oil was administered once each by oral gavage in rats of both sexes. The rats were starved for 16 hours before and 2 hours after the administration of the test substance. The animals were observed for 14 days after the dosing for the product related toxic symptoms and mortality. Necropsy was carried out in animals died during the observation period. At the end of the observation period the surviving test animals were sacrificed, dissected and examined for gross pathological abnormality, if any.
LD₅₀ in rats

Ten rats (5 male and 5 female) was allocated to treatment as follows.

| Group No. | No. of rats per sex | Dose (mg/kg) |
|---|---|---|
| 1 | 5 | 5000 |

### RESULTS

LD₅₀ in rats

| Group No. | Dose (mg/kg) | No. of animals died | Mortality |
|---|---|---|---|
| | | No. of animals treated | |
| 1 | 5000 | 0/10 | 0 |

| | | | |
|---|---|---|---|
| Symptoms: Group 1 - Salivation in all animals and polyurea in two animals were observed with onset at 2 hours after the treatment. All animals survived through the study period of 14 days and were free of intoxicating signs 24 hours after the treatment. Necropsy findings: No significant changes could be seen with naked eye. | | | |

LD₅₀: Greater than 5000 mg/kg.

**Mortality Table**

| | | | | | | | | | | | | | | | | | | | |
|---|---|---|---|---|---|---|---|---|---|---|---|---|---|---|---|---|---|---|---|
| Gr. No | Dose mg/kg | Mortality | | | | | | | | | | | | | | | | | |
| | | Hours | | | | Day | | | | | | | | | | | | | No. of animals died |
| | | 2 | 4 | 6 | 24 | 2 | 3 | 4 | 5 | 6 | 7 | 8 | 9 | 10 | 11 | 12 | 13 | 14 | No. of animals treated |
| 1 | 5000 | 0 | 0 | 0 | 0 | 0 | 0 | 0 | 0 | 0 | 0 | 0 | 0 | 0 | 0 | 0 | 0 | 0 | 0/10 |

### PROJECT NO. 4953

### SUMMARY

The study was designed to determine the primary skin irritation potential of Colour Removed Curcumin supplied by **Sami Chemicals and Extracts Ltd., Bangalore.** The test substance in the amount of 500 mg was applied to shorn back skin both intact and abraded site of three rabbits per sex. Each site of application was carefully observed and the reaction evaluated according to Draize's method at 24 and 72 hours.
**Colour Removed Curcumin** did not cause irritation to skin in rabbits.
Primary skin irritation score = 0.00.

### MATERIALS AND METHODS

### TEST SUBSTANCE:

| | |
|---|---|
| Sponsor : | **Sami Chemicals and Extracts Ltd., Bangalore.** |
| Label on Sample : | **Colour Removed Curcumin** Batch No.- CRC/65 A R No. - BL8C 0445 |
| Description : | Creamy white crystalline powder |
| Identification : | IR Spectrum : Complies |
| Solubility : | Soluble in methanol |
| Purity by HPLC : | 99.0% |

### TEST SYSTEM

- Species:: Rabbit
- Strain:: New Zealand White
- Source:: Serum Institute of India Ltd., Pune.
- Sex:: Male and Female
- Age:: Healthy adult
- Weight range:: 2.00 to 2.40 kg
- No. of animals per dose:: 3 per sex
- Randomization:: Randomly selected in groups of three of like sex at the time of initiating the study.
- Acclimation:: Seven days period to treatment.
- Identification of animals:: : By cage number and individual marking on ear.
- Diet:: Pelleted feed supplied by Nav Maharashtra Chakan Oil Mills Ltd., Pune.
- Water:: Aquaguard pure water in glass bottles *ad libitum.*
- Management:: The rabbits were housed individually each, in stainless steel cages provided with stainless steel mesh bottom. The temperature was maintained between 20 & 23 °C and relative humidity between 50 and 60%; 12 hours each of dark and light cycle was maintained.
- Route:: Dermal
- Dose volume:: 500 mg/site.

### EXPERIMENTAL PROCEDURE

Three rabbits per sex were used for this study. The hair on both the sides were removed by electric clippers one day before the treatment. The test substance in the amount of 500 mg was applied to the intact and abraded skin sites of each test animal. Care was taken to note that the abrasions penetrate the Stratum corneum but not the dermis. A gauze patch was secured over each treated area by means of adhesive tape. The animals were housed singly with plastic collar around their necks for 24 hours, in order to avoid the ingestion of test substance. The patch and unabsorbed test substance was removed after 24 hours. Each site of application was carefully observed and reaction evaluated according to Draize's method at 24 and 72 hours.

### Draize Evaluation of Skin Reactions -

Grading of skin reaction

| 1. | Erythema and Eschar Formation | |
|---|---|---|
| | No erythema | 0 |
| | Very slight erythema (barely perceptible) | 1 |
| | Well defined erythema | 2 |
| | Moderate to severe erythema Severe erythema (beet redness) to slight | 3 |
| | eschar formation (injuries in depth) | 4 |
| | Total maximum possible erythema score: 4 | |

| 2. | Edema Formation | |
|---|---|---|
| | No edema | 0 |
| | Very slight edema (barely perceptible) | 1 |
| | Slight edema (edges of area well defined by definite raising) | 2 |
| | Moderate edema (raised approximately 1 mm) Severe edema (raised more than 1 mm and extending | 3 |
| | beyond area of exposure | 4 |
| | Total maximum possible edema score: 4 | |

### RESULTS

Summary of individual dermal irritation score following application of Colour Removed Curcumin on rabbits.

| RABBIT No. | SEX | ABRADED | | | | INTACT | | | |
|---|---|---|---|---|---|---|---|---|---|
| | | 24 Hours | | 72 Hours | | 24 Hours | | 72 Hours | |
| | | Erythema | Oedema | Erythema | Oedema | Erythema | Oedema | Erythema | Oedema |
| 1 | Male | 0 | 0 | 0 | 0 | 0 | 0 | 0 | 0 |
| 2 | Male | 0 | 0 | 0 | 0 | 0 | 0 | 0 | 0 |
| 3 | Male | 0 | 0 | 0 | 0 | 0 | 0 | 0 | 0 |
| 4 | Female | 0 | 0 | 0 | 0 | 0 | 0 | 0 | 0 |
| 5 | Female | 0 | 0 | 0 | 0 | 0 | 0 | 0 | 0 |
| 6 | Female | 0 | 0 | 0 | 0 | 0 | 0 | 0 | 0 |
| Total | | 0 | 0 | 0 | 0 | 0 | 0 | 0 | 0 |
| Mean | | 0 | 0 | 0 | 0 | 0 | 0 | 0 | 0 |
| Grand Total | | 0 | | | | | | | |

Primary skin irritation score 0.00 / 4 = 0.00

### CONCLUSION

It is concluded based on the present study that of the test substance **Colour Removed Curcumin** supplied by Sami **Chemicals and Extracts Ltd., Bangalore** did not cause irritation to skin in rabbits.
Primary skin irritation score : 0.00.

Scavenging effect (%) of curcumin and its derivatives on 1, 1-diphenyl-2-picrylhydrazyl radical.

| Compound | | concentration (µg/ml) | |
|---|---|---|---|
| | 32.8 | 16.4 | 8.2 |
| curcuminoids sample1 | 88.09 ± 0.4 | 83.04 ± 1.5 | 51.20 ± 0.9 |
| curcuminoids sample2 | 88.71 ± 0.4 | 81.51 ± 0.1 | 53.60 ± 1.6 |
| tetrahydrocurcumin | 88.17 ± 0.9 | 83.00 ± 0.7 | 65.76 ± 0.9 |
| tetrahydrocurcumin(NTC) | 87.39 ± 0.4 | 82.30 ± 1.4 | 68.61 ± 0.8 |
| tetrahydrocurcumin(STC) | 84.07 ± 2.0 | 77.13 ± 0.8 | 56.53 ± 0.7 |

| | | | |
|---|---|---|---|
| * NTC: natural; STC: synthetic. | | | |

Effect of topical application of curcumin and tetrahydrocurcumin on ultraviolet B light-induced formation of thymine dimers in mouse epidermis

| Treatment | Number of per group | Percent of thymine dimer cells | Percent of protection |
|---|---|---|---|
| 1. No UVB | 3 | 0 | - |
| 2. Acetone + UVB | 7 | 8.9 ± 0.02 | 0 |
| | | | |
| 3. THC (10 µmol) + UVB | 6 | 7.3 ± 0.03 | 24.7 |
| 4. THC (20 µmol) + UVB | 6 | 1.6 ± 1.30 | 82.0 |
| | | | |
| 5. Curcumin (10 µmol) + UVB | 5 | 1.6 ± 0.01 | 82.0 |
| 6. Curcumin (20 µmol) + UVB | 5 | 1.2 ± 1.69 | 86.5 |

Female SKH-1 mice (8-9 weeks old) were treated topically with 100 µl acetone, or test compound in 100 µl acetone. Five minutes later, the mice were irradiated with a single dose of UVB (30 mJ/cm²). The mice were killed one hour later, and the skin samples were stored in a 10% formalin-phosphate buffer for thymine dimers assay.

Effect of UVB-induced formation of skin sunburn cells in mouse epidermis

| Treatment | Number of per group | Percent of sunburn cells | percent of inhibition |
|---|---|---|---|
| No UVB | 5 | 0.1 | - |
| | | | |
| Acetone + UVB (60 mJ/cm²) | 5 | 3 | - |
| | | | |
| DBM + (10 µmol) | 5 | 1.2 | 60 |
| DBM + (20 µmol) | 5 | 0.9 | 70 |
| | | | |
| Tetrahydrocurcumin (10 µmol) + UVB | 5 | 1.6 | 47 |
| Tetrahydrocurcumin (20 µmol) + UVB | 5 | 1.4 | 53 |

Female Sencar mice were irradiated with UVB (60 mJ/cm²). The mice were treated topically with 100µl acetone or inhibitor in 100 µl acetone at 5 min before the UVB irradiation. Eight hours later, the mice were killed and skin samples were removed and kept in 10% formalin phosphate buffer for sunburn cells assay.

## Claims

1. Use of an effective amount of a mixture of tetrahydrocurcuminoids for the manufacture of a medicament for treating melanoma.

2. Use of an effective amount of a mixture of tetrahydrocurcuminoids for the manufacture of a medicament for the treatment of age spots due to lipofuscin formation.

## Patentansprüche

1. Verwendung einer wirksamen Menge einer Mischung von Tetrahydrocurcuminoiden zur Herstellung eines Medikaments zur Behandlung von Melanomen.

2. Verwendung einer wirksamen Menge einer Mischung von Tetrahydrocurcuminoiden zur Herstellung eines Medikaments zur Behandlung von Altersflecken aufgrund der Bildung von Lipofuscin.

## Revendications

1. Utilisation d'une quantité efficace d'un mélange de tétrahydrocurcuminoïdes pour la fabrication d'un médicament destiné à traiter le mélanome.

2. Utilisation d'une quantité efficace d'un mélange de tétrahydrocurcuminoïdes pour la fabrication d'un médicament destiné à traiter les tâches de vieillesse dues à la formation de lipofuscine.
